# EUROPEAN PATENT SPECIFICATION

(11) **EP 2 105 088 B1**
(45) Date of publication and mention of the grant of the patent: **29.06.2011**
(21) Application number: 09004174.0
(22) Date of filing: 24.03.2009
(51) Int. Cl.: A61B 3/18, G09B 23/28

(54) **Simulation apparatus, simulation program, and recording medium recorded with simulation program**
Simulationsvorrichtung, Simulationsprogramm und mit dem Aufzeichnungsmedium aufgezeichnetes Simulationsprogramm
Appareil de simulation, programme de simulation et support d'enregistrement sur lequel un programme de simulation est enregistré

(30) Priority: 26.03.2008 JP 2008080287; 06.01.2009 JP 2009000928
(43) Date of publication of application: 30.09.2009
(73) Proprietor: Seiko Epson Corporation, Shinjuku-ku Tokyo (JP)
(72) Inventor: Shinohara, Toshihide, Suwa-shi Nagano-ken 392-8502 (JP); Kaga, Tadashi, Suwa-shi Nagano-ken 392-8502 (JP)
(74) Representative: HOFFMANN EITLE

(56) References cited:
- EP-A- 1 018 691
- WO-A-01/62139
- US-A1- 2004 174 499

## Description

### BACKGROUND

### 1. Technical Field

The present invention relates to a simulation apparatus for simulating a way of being seen through a lens, a simulation program, and a recording medium recorded with a simulation program.

### 2. Related Art

Simulation apparatuses that simulate a way of being seen through a lens, such as a ophthalmic lens, on a viewing area of a display device are known (JP-A-2000-107129 and JP-A-2002-45336). In JP-A-2000-107129 and JP-A-2002-45336, a way of being seen with sway, distortion, blur when a progressive power lens and other lenses are worn is simulated.

In the known examples disclosed in JP-A-2000-107129 and JP-A-2002-45336, visual motion characteristics of a person (hereinafter, referred to as a 'head mover') who mainly moves the head when turning the eyes to an object and a person (hereinafter, referred to as an 'eye mover') who mainly moves the eyes are not considered.

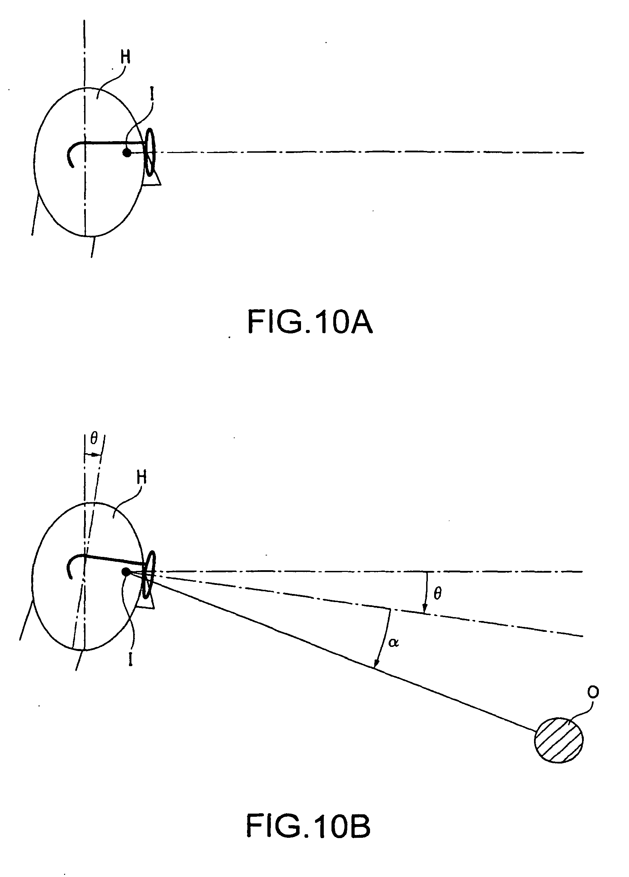

A visual motion characteristic of a lens user will be described on the basis of Figs. 10A to 11B.

Figs. 10A and 10B are schematic views illustrating the motion of head and eyes of a lens user in the vertical direction (up and down direction). Fig. 10A shows a state where a lens user naturally takes a horizontal look, and Fig. 10B shows a state where the lens user takes a look at an object O positioned at the lower side. Compared with the state shown in Fig. 10A, a frontward tilt angle θ of a head H of the lens user and a rotation angle α of an eye I exist in the state shown in Fig. 10B.

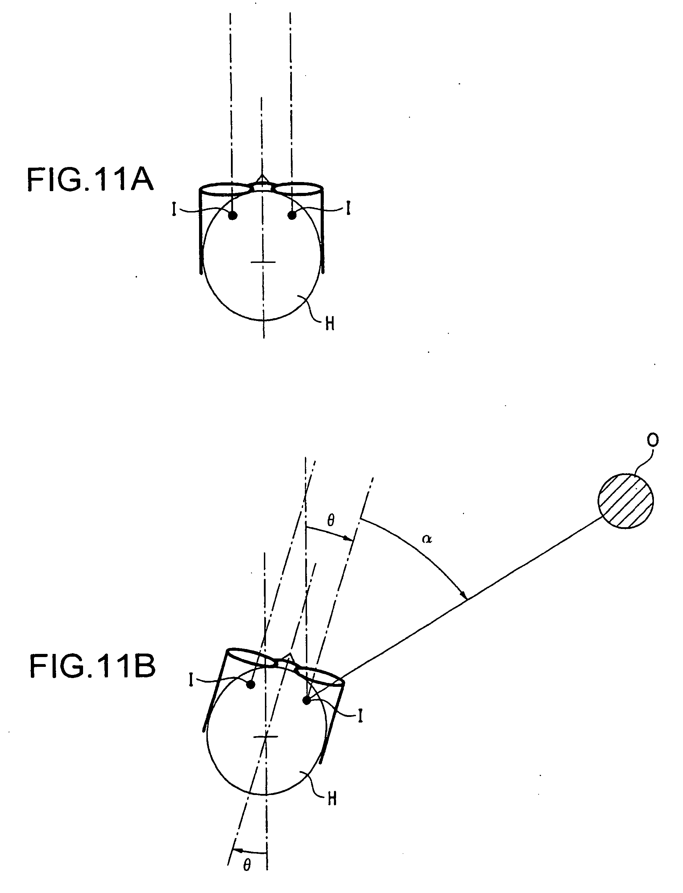

Figs. 11A and 11B are views illustrating the motion of head and eyes in the horizontal direction (lateral direction). Fig. 11A shows a state where the lens user takes a look at the front side within a horizontal plane, and Fig. 11B shows a state where the lens user takes a look at the object O positioned on the right side within the horizontal plane. Compared with the state shown in Fig. 11A, a rotation angle θ of the head H of the lens user in the lateral direction and a rotation angle α of the eye I exist in the state shown in Fig. 11B.

A lens user in the case when a ratio of the motion θ of the head and the motion α of the eye is set to be θ >> α is a head mover, and a lens user in the case when the ratio is set to be θ << α is an eye mover. People are not cleanly classified into head movers and eye movers, but intermediate head movers and eye movers also exist. The visual motion characteristics of lens users called head movers and eye movers should be taken into consideration when designing progressive power lenses.

The visual motion characteristics of the head movers and the eye movers are habits that people perform unconsciously. On the other hand, it is also necessary to know that people generally position their faces to face an observation object when watching the observation object, that is, people act as perfect head movers in the case.

Generally, a progressive power lens has: a far vision portion for seeing a far place positioned at the upper side from the approximate half of the lens; a near vision portion that is spaced apart from the far vision portion and is used to see a near place positioned at the lower side; and a progressive portion that is located between the far vision portion and the near vision portion and in which the refractive power progressively changes. The progressive power lens has optical distortion (for example, astigmatism or distortion aberration) on a side portion of the lens, mainly on sides of the progressive portion and near vision portion. In such a portion, an image is seen in a blurred or distorted state. The astigmatism is a cause of image blur. When the astigmatism increases, an object is not clearly seen in the portion due to the blur. In general, a portion where the astigmatism is 0.5 diopter or less can be used with little feeling of blur. For this reason, the portion where the astigmatism is 0.5 diopter or less is called a 'clearly visible region'. There are types in design of a progressive power lens. Design corresponding to a case where clearly visible regions of far vision portion and near vision portion are wide is referred to as hard design. On the contrary, design corresponding to a case where clearly visible regions of far vision portion and near vision portion are narrow is referred to as soft design.

The hard design is advantageous in that easy viewing is realized since clearly visible regions of the far vision portion and near vision portion are wide but disadvantageous in that large astigmatism occurs on the side of the progressive portion and unpleasant sway is large in moving the head since a clearly visible region of the progressive portion is narrow.

The soft design is disadvantageous in that the clearly visible regions of the far vision portion and near vision portion are narrow but advantageous in that the clearly visible region of the progressive portion is wide and the sway is small since distortion or astigmatism of the progressive portion is small.

A progressive power lens of soft design is suitable for a lens user who is a head mover, and a progressive power lens of hard design is suitable for a lens user who is an eye mover.

There is a known example in which the visual motion characteristics of head movers and eye movers are taken into consideration. This known example is a method of performing classification from the point of view of the motion of the head and eyes and recommending a design of a ophthalmic lens, especially a progressive power lens on the basis of the classification (JP-T-2003-523244).

In the known example disclosed in JP-T-2003-523244, however, a unit that checks whether or not the design is suitable for a lens user is not disclosed even though it is possible to recommend a ophthalmic lens for the eye mover or a ophthalmic lens for the head mover.

As a method of checking whether or not the lens is suitable for the lens user, a method of having a wearing experience by preparing a representative lens for each design called a trial lens or a wearing test lens and placing the trial lens or the wearing test lens in an trial frame, in which an trial lens for correcting the refractive error of the lens user is placed, so as to overlap the trial lens is considered.

However, in this method, it is practically difficult to meet all cases in which degrees of head movers or eye movers are different even if a pseudo experience using a trial lens designed for a typical head mover or eye mover is possible.

### SUMMARY

An advantage of some aspects of the invention is that it provides a simulation apparatus, a simulation program, and a recording medium recorded with a simulation program capable of simulating a way of being seen through a lens in which visual motion characteristics regarding the motion of the head and eyes when a lens user takes a look are considered.

According to an aspect of the invention, there is provided a simulation apparatus including: a design data acquiring unit that acquires lens design data; a lens design unit that designs a lens on the basis of the lens design data; an image data acquiring unit that acquires image data corresponding to a lens user's visual sense; a visual motion characteristic data acquiring unit that acquires visual motion characteristic data regarding the motion of head and eyes when the lens user moves the eyes to various observation objects; an image processing unit that creates processing image data which is the image data seen through the design lens designed by the lens design unit; an image moving unit that moves the processing image data created by the image processing unit; an image data control unit that controls a movement amount of the processing image data, which is moved by the image moving unit, on the basis of the visual motion characteristic data; and a display unit that displays the processing image data.

According to the aspect of the invention, the lens design unit of the simulation apparatus designs a lens on the basis of the lens design data acquired by the design data acquiring unit. In this case, the shape of the edge of a lens designed may be a shape before lens machining (generally, circular shape) or may be a machined lens shape. The image data acquiring unit acquires image data corresponding to the lens user's visual sense. The visual motion characteristic data acquiring unit acquires visual motion characteristic data regarding the motion of head and eyes when the lens user moves the eyes to various observation objects. Here, the visual motion characteristic data may be evaluated, for example, by attaching to the lens user's head a device for detecting the position of the head and causing the lens user to repeatedly see upper and lower and left and right objects to thereby detect the motion of the head and eyes.

In addition, the image processing unit creates processing image data expressing a state where the image data is seen through a processed lens obtained by machining the design lens designed by the lens design unit, and the processing image data is displayed on the display unit.

In addition, when the image data control unit receives the visual motion characteristic data acquired in the visual motion characteristic data acquiring unit, the image data control unit transmits a signal to the image moving unit and the image moving unit moves the processing image data by a predetermined movement amount. This moved processing image data is displayed on the display unit.

On the display unit, both processing image data before being moved by the image moving unit and processing image data after being moved by the image moving unit are displayed.

In the aspect of the invention, since the processing image data is made to move according to visual motion characteristics regarding the motion of the head and eyes of the lens user, a way of being seen through a lens corresponding to the visual motion characteristic of each lens user can be simulated.

In the simulation apparatus according to the aspect of the invention, preferably, the visual motion characteristic data is a ratio between an amount of rotation of the head and an amount of rotation of the eyes when the lens user moves the eyes to various observation objects.

In the aspect of the invention, since the visual motion characteristic of the lens user is mainly set by the ratio between the amount of rotation of the head and the amount of rotation of the eyes, a way of being seen through a lens can be simulated on the basis of a visual motion characteristic of each lens user.

In the simulation apparatus according to the aspect of the invention, preferably, the image data control unit has a standard mode in which the processing image data is made to move according to the amount of rotation of the head acquired in the visual motion characteristic data acquiring unit.

In the aspect of the invention, a way of being seen through a lens can be simulated on the basis of a visual motion characteristic of each lens user.

In the simulation apparatus according to the aspect of the invention, preferably, the image data control unit has a watch mode in which the processing image data is made to move according to a total value of the amount of rotation of the head and the amount of rotation of the eyes acquired in the visual motion characteristic data acquiring unit.

In the aspect of the invention, a way of being seen when the lens user moves the eyes to watch an object can be simulated irrespective of the head mover or the eye mover.

In the simulation apparatus according to the aspect of the invention, preferably, the lens is a progressive power lens including a far vision portion for seeing a far place, a near vision portion for seeing a near place, and a progressive portion that is located between the far vision portion and the near vision portion and in which the refractive power progressively changes.

In the simulation apparatus according to the aspect of the invention, a way of being seen through a progressive power lens can be checked according to a visual motion characteristic of each lens user.

Furthermore, in the aspect of the invention, preferably, the lens design unit designs two kinds of lenses including a lens with the wide far vision portion and / or the wide near vision portion and a lens with the narrow far vision portion and / or the narrow near vision portion.

In the aspect of the invention configured as described above, the lens user can check through the simulation apparatus which one of a progressive power lens of hard design, which is characterized in that a clearly visible region of a far vision portion or near vision portion is wide, and a progressive power lens of soft design, which is characterized in that a clearly visible region of a far vision portion or near vision portion is narrow, is suitable.

According to another aspect of the invention, there is provided a simulation program causing a calculation unit to function as the simulation apparatus described above.

According to the aspect of the invention, the calculation unit is made to operate as the above-described simulation apparatus by the simulation program. Accordingly, the lens user can easily compare the way of being seen through lenses according to visual motion characteristics.

According to still another aspect of the invention, there is provided a recording medium recorded with a simulation program in which the above-described simulation program is recorded to be readable by the calculation unit.

According to the aspect of the invention, the above-described simulation program is recorded in the recording medium so as to be readable by the calculation unit. Accordingly, by causing the calculation unit to read the stimulation program from the recording medium, the above-described simulation program can be executed in the calculation unit.

### BRIEF DESCRIPTION OF THE DRAWINGS

The invention will be described with reference to the accompanying drawings, wherein like numbers reference like elements.

Fig. 1 is a schematic view illustrating the configuration of a simulation apparatus according to an embodiment of the invention.

Fig. 2 is a flow chart illustrating an operation of the simulation apparatus according to the embodiment.

Fig. 3 is a flow chart illustrating the procedure of an image moving process.

Fig. 4 is a view illustrating an original image of simulation.

Fig. 5 is a view illustrating image data of the simulation apparatus.

Figs. 6A and 6B are views illustrating an eye movement position designating process.

Figs. 7A to 7C are views illustrating a process of moving image data.

Figs. 8A to 8C are views illustrating a case where a progressive power lens of soft design is displayed.

Figs. 9A to 9C are views illustrating a case where a progressive power lens of hard design is displayed.

Figs. 10A and 10B are schematic views illustrating the motion of head and eyes of a lens user in the vertical direction (up and down direction).

Figs. 11A and 11B are schematic views illustrating the motion of head and eyes of a lens user in the horizontal direction (lateral direction).

### DESCRIPTION OF EXEMPLARY EMBODIMENTS

Hereinafter, a simulation apparatus according to an embodiment of the invention will be described with reference to the accompanying drawings.

### Configuration of a simulation apparatus

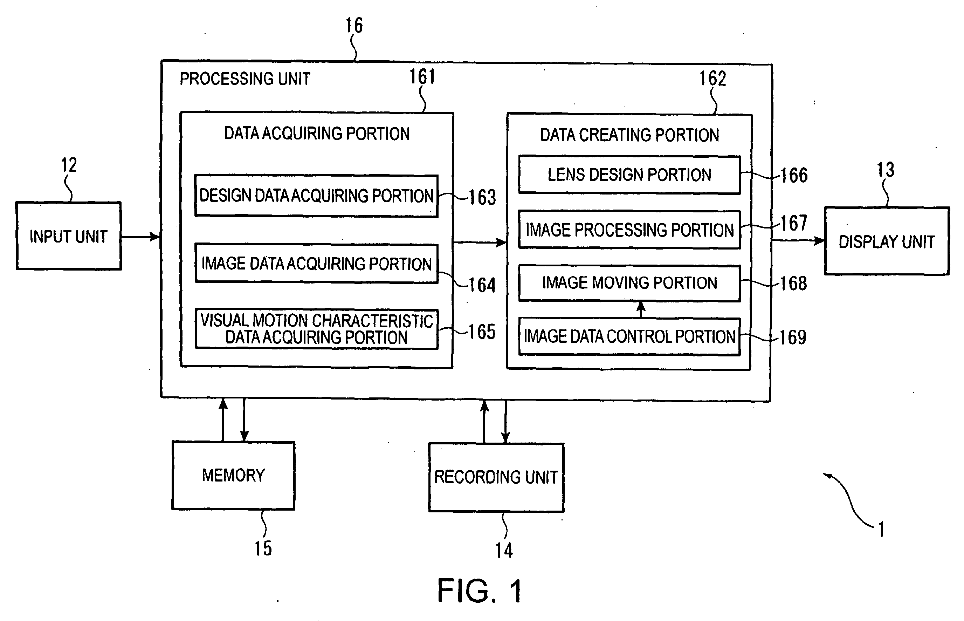

Fig. 1 is a schematic view illustrating the configuration of a simulation apparatus 1 according to the present embodiment.

The simulation apparatus 1 is set in a ophthalmic lens shop, for example.

Moreover, although a personal computer is illustrated as the simulation apparatus 1 in the present embodiment, the invention is not limited thereto. For example, other calculation units, such as portable phones, may also be used.

As shown in Fig. 1, the simulation apparatus 1 includes an input unit 12, a display unit 13 as a display means, a recording unit 14 as an image recording means, a memory 15, and a processing unit 16.

The input unit 12 is a keyboard or a mouse, for example, and has various operation buttons (not shown) or operation knobs (not shown) used for input operations.

The input operations of the operation buttons or operation knobs are setting input of setting details, such as setting of details of an operation of the simulation apparatus 1 and setting of information stored in the simulation apparatus 1.

In addition, by the input operation of setting details, the input unit 12 suitably outputs a signal corresponding to the setting details to the processing unit 16 to thereby perform setting input.

In addition, the input operations are not limited to operations of the operation buttons or operation knobs. For example, setting input of various setting details may also be performed by an input operation using a touch panel provided in the display unit 13 or an input operation using a voice.

The display unit 13 is controlled by the processing unit 16 and performs screen display of a signal of image information, which is input from the processing unit 16, in a display area (not shown).

Examples of the display unit 13 include a liquid crystal panel, an organic EL (electro luminescence) panel, a PDP (plasma display panel), a CRT (cathode-ray tube), an FED (field emission display), an electrophoretic display panel.

The recording unit 14 stores various kinds of data, such as customer data, an original image to be simulated, simulated image data, and visual motion characteristic data, that is, readably stores the data.

The recording unit 14 may be a drive that readably stores data in recording media, such as an HD (hard disk), a DVD (digital versatile disc), an optical disk, and a memory card, or may be configured to include a driver.

Here, customer data is data on prescription of lenses ordered by a customer who is a lens user. The customer data is one data obtained by associating customer ID data, prescription data, and lens shape design data with each other.

In addition, lens design data of the invention is built by the prescription data and the lens shape design data.

The customer ID data is unique information for specifying customer data and is also information set for every customer data. Examples of the customer data may include a customer number set for every customer and customer personal information on the name and the like of a customer.

The prescription data is data on a visual sense of a customer or prescription of a lens of customer data specified as customer ID data. Visual sense data on the customer's visual sense, lens prescription data on prescription of a lens to design, and the like are recorded in the prescription data.

Information on the visual sense in the naked eyes of a customer, such as customer's eyesight or astigmatism, is recorded in visual sense data. Moreover, data regarding the power of a lens, addition power, spherical power, astigmatic power, astigmatism axis, prism power, near vision portion offset amount, and the like is recorded in the lens prescription data.

The lens shape design data is data on the shape of a lens. For example, the Abbe number or refractive index of a lens material, coordinate value data of a lens refracting surface (front surface, rear surface), thickness data such as a thickness of the center of a lens, and data on a design parameter such as a progressive zone length are recorded. In addition, data on a refractive operation (for example, refractive power and prism action) at each point on a lens may also be included.

The memory 15 stores the setting details, voice information or image information, and the like input by an operation using the input unit 12 so as to be able to be suitably read. Furthermore, the memory 15 stores various programs loaded onto the OS (operating system) that controls the entire operation of the simulation apparatus 1. In addition, the memory 15 may be a drive that readably stores programs in recording media, such as an HD, a DVD, and an optical disk, or may be configured to include a driver.

The processing unit 16 has various input and output ports (not shown), for example, a key input port to which the input unit 12 is connected, a display port to which the display unit 13 is connected, a memory port to which the recording unit 14 is connected, and a memory port to which the memory 15 is connected.

In addition, the processing unit 16 includes a data acquiring portion 161 and a data creating portion 162 as various programs as shown in Fig. 1.

The data acquiring portion 161 recognizes an input signal input by a user's input operation using the input unit 12 and acquires various kinds of data based on the input signal. In addition, the data acquiring portion 161 acquires various kinds of data from the recording unit 14.

The data acquiring portion 161 has a design data acquiring portion 163 that acquires lens design data, an image data acquiring portion 164 that acquires an image such as image data corresponding to the customer's visual sense, and a visual motion characteristic data acquiring portion 165 that acquires visual motion characteristic data regarding the motion of the head and eyes when a lens user moves his or her eyes to various observation objects.

The data creating portion 162 creates new data from the various data acquired in the data acquiring portion 161.

The data creating portion 162 has a lens design portion 166 that designs a lens (design lens) before lens machining based on lens design data, an image processing portion 167 that creates processing image data expressing a state where image data is seen through a processed lens obtained by performing lens machining on the design lens, an image moving portion 168 that moves the processing image data created by the image processing portion 167, and an image data control portion 169 that controls the movement amount of the processing image data, which is moved by the image moving portion 168, on the basis of the visual motion characteristic data acquired in the visual motion characteristic data acquiring portion 165.

The image data control portion 169 has an eye movement position designating mode for designating the eye movement position among the visual motion characteristic data acquired in the visual motion characteristic data acquiring portion 165, a standard mode for moving the processing image data according to the amount of rotation of the eyes acquired in the visual motion characteristic data acquiring portion 165, and a watch mode for moving the processing image data according to the total value of the amount of rotation of the head and the amount of rotation of the eyes acquired in the visual motion characteristic data acquiring portion 165. These modes are switched by a signal from the input unit 12.

### Operation of a simulation apparatus

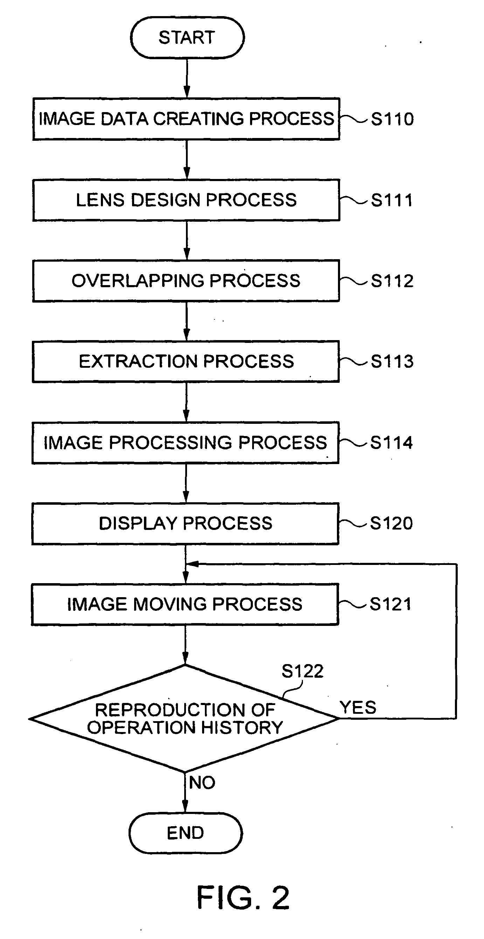

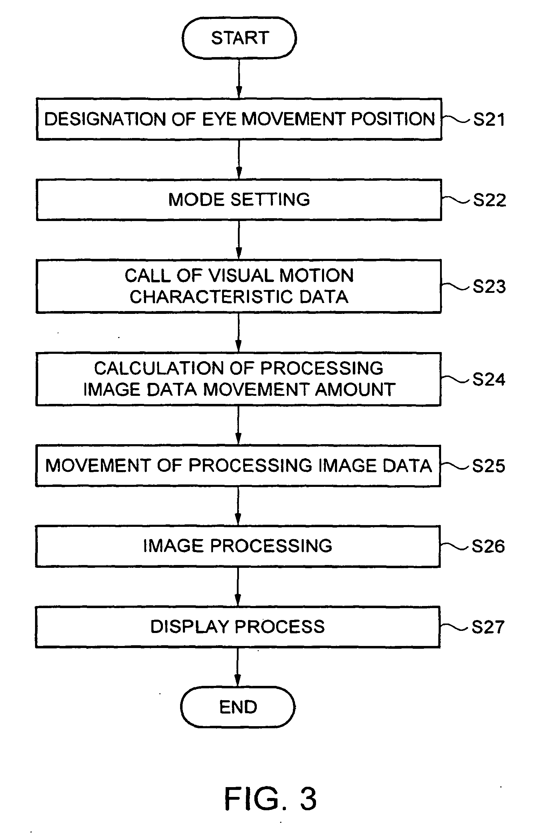

Hereinafter, an operation of the simulation apparatus 1 will be described with reference to Figs. 2 and 3.

Moreover, in the present embodiment, a lens is a progressive power lens that has a far vision portion for seeing a far place, a near vision portion for seeing a near place, and a progressive portion which is located between the far vision portion and the near vision portion and in which the refractive power progressively changes.

First, visual motion characteristic data regarding the motion of the head and eyes when a lens user sees a moving observation object is obtained by using a separate apparatus. As this apparatus, the 'Varilux VisionPrint System' of Nikon-Essilor Co., Ltd. may be used, for example.

The visual motion characteristic data may be evaluated by attaching to the lens user's head a device for detecting the position of the head and causing the lens user to repeatedly see upper and lower and left and right objects to thereby detect the motion of the head and eyes.

For example, a rotation angle θ of the head and a rotation angle α of the eyes are quantified as θ : α = 4 : 6. The visual motion characteristic data is stored in the recording unit 14 by operating the input unit 12.

Fig. 2 is a flow chart illustrating an operation of the simulation apparatus according to the embodiment of the invention.

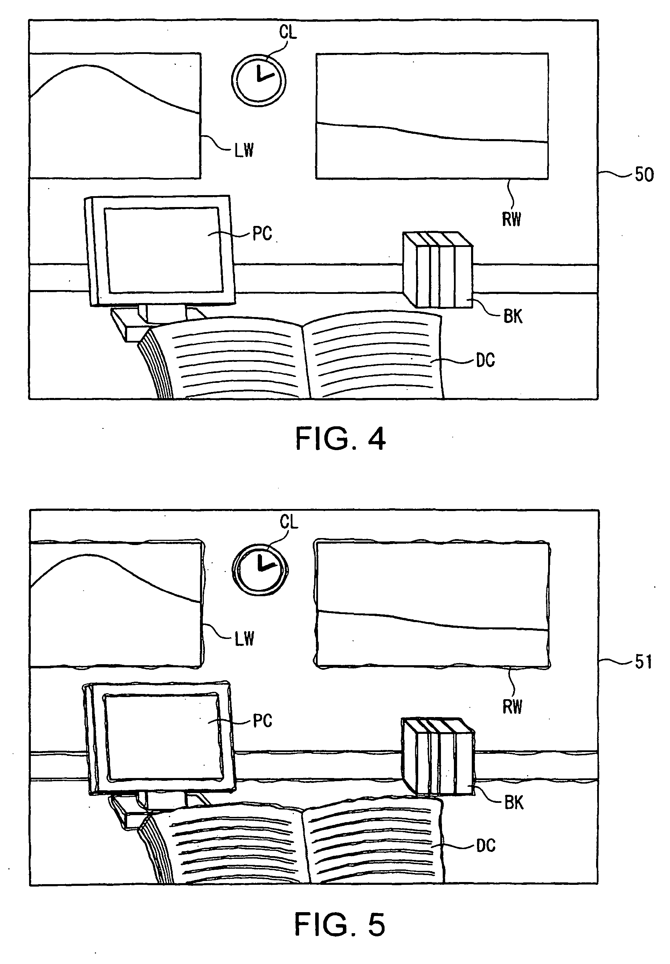

First, in an image data creating process S110, the data creating portion 162 reads an original image 50 (refer to Fig. 4) of simulation and visual sense data of a customer from the recording unit 14. The original image 50 is an example of a virtual observation space and is obtained by imaging of an office. Although scenes seen through left and right windows LW and RW, a clock CL on a wall, a personal computer PC on a desk, a document DC at hand, and books BK standing on a desk are expressed in a planar manner, these objects are actually disposed as three-dimensional data in a three-dimensional space. In addition, the original image 50 is only an example, but an image including other scenes, a man, or an object may also be used as the original image 50 without being limited to the above-described original image 50.

Then, the data creating portion 162 performs image processing on the original image 50 to obtain a state corresponding to visual sense data of a customer, that is, the eyesight or astigmatic power, heterophoria, convergence power, and the like of the naked eyes of the customer.

For example, image data 51 (refer to Fig. 5) corresponding to the customer's visual sense is created by blurring the outline of an image, forming multiple outlines, spreading a color, or distorting the original image 50. The created image data 51 is recorded in the recording unit 14.

Then, in a lens design process S111, the design data acquiring portion 163 reads lens design data from the recording unit 14 and the lens design portion 166 designs a lens (design lens) before lens machining based on the read lens design data. In addition, an edge shape may be designed in the lens cut into a shape after lens machining.

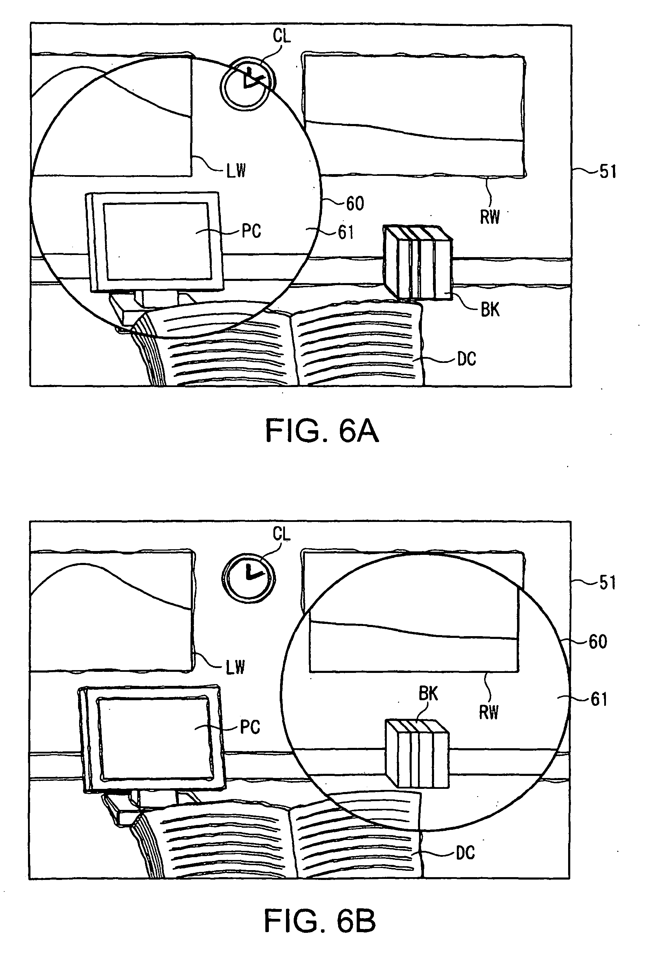

In an overlapping process S112, the image data acquiring portion 164 reads the image data 51 from the recording unit 14, and the image processing portion 167 makes lens image data 60 on an image of the design lens overlap the read image data 51 as shown in Fig. 6A. Although the lens image data 60 is circular in this example, it may be possible to consider an image seen through a design lens before lens machining or an image seen through a design lens processed to be of a circular lens type.

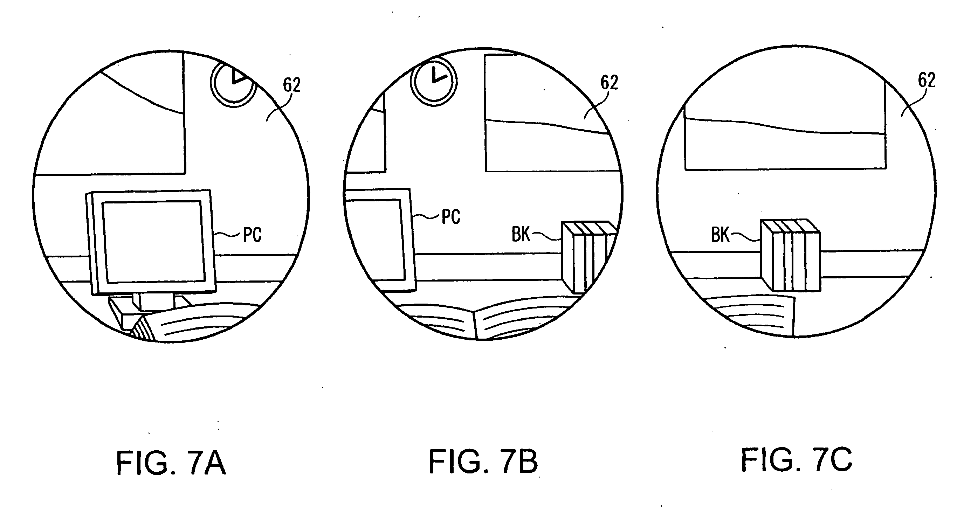

Subsequently, in an extraction process S113, the image processing portion 167 extracts an overlapping portion in which the image data 51 and the lens image data 60 overlap each other as shown in Fig. 7A. At this time, the extracted range is adjusted according to the prescription power of a lens. That is, in the case of a lens corresponding to minus prescription, it is necessary to perform extraction in a larger range than the edge of the lens since a transmission image is reduced due to refraction of the lens. In the case of a lens corresponding to plus prescription, it is preferable to perform extraction in a smaller range than the edge of the lens. In any case, it is preferable to extract the overlapping portion largely with some allowances, perform image processing to be described later, and then cut a portion beyond the edge of the lens.

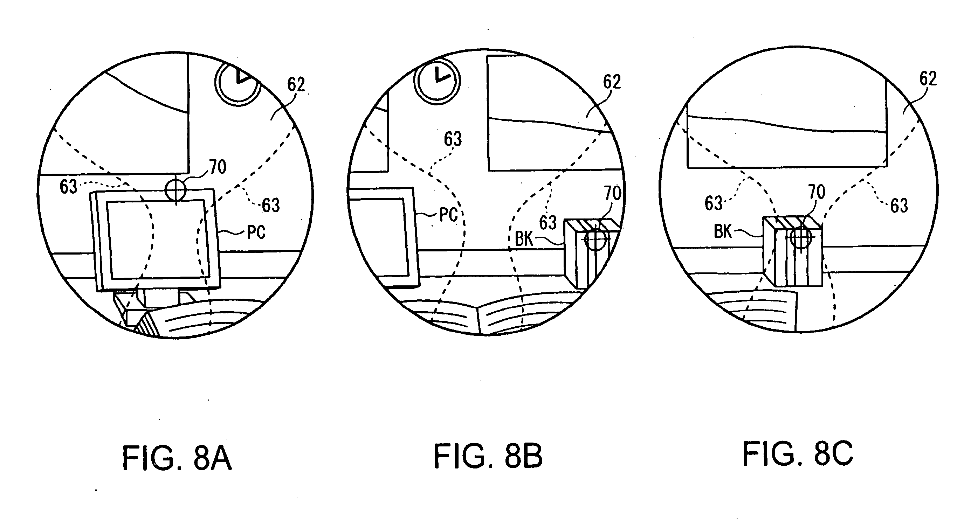

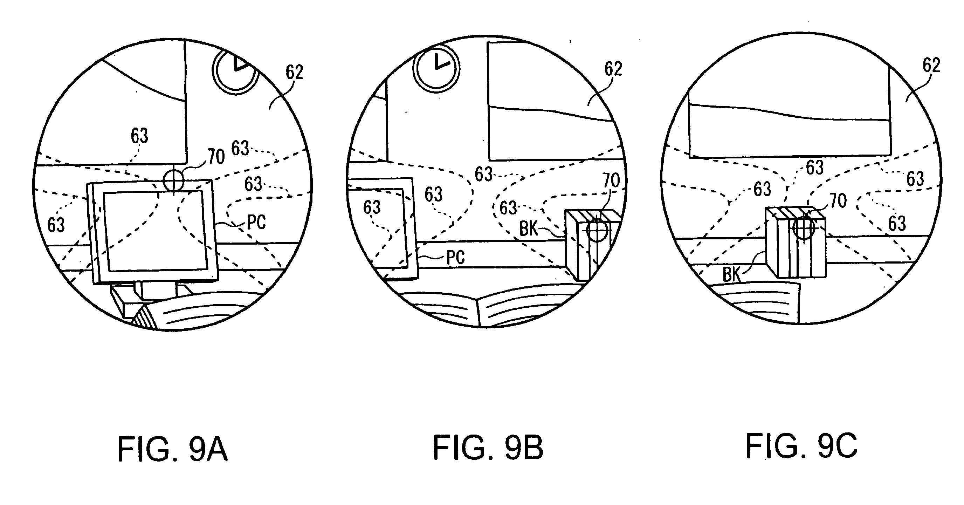

In an image processing process S114, the image processing portion 167 obtains processing image data 62 by performing image processing on an overlapping portion 61 to obtain a state where the image data 51 is seen through the design lens (refer to Figs. 8A and 9A). The obtained processing image data 62 is recorded in the recording unit 14.

In a display process S120, the processing image data 62 recorded in the recording unit 14 is called to be displayed on the display unit 13.

In the image displayed on the display unit 13, a broken line 63 is displayed on the lens as shown in Figs. 8A and 9A. The broken line 63 shows the boundary between the far vision portion, the progressive portion, and the near vision portion of the design lens, which is the progressive power lens, and a portion (left and right of a lower portion of the lens) that cannot be used due to optical distortion. The processing image data 62 is image processed such that the lens image data 60 is differently seen with the broken line 63 as a boundary. In addition, in Figs. 8A to 9C, enlargement of an image in the near vision portion or distortion of an image in the progressive portion, which is a viewing feature in a progressive power lens, is omitted. In addition, the broken line 63 is not clearly recognized in actual use of the progressive power lens. Accordingly, the broken line 63 is not necessarily required on the simulation. However, a result of the simulation can be understood more clearly by setting the broken line 63.

After the display process S120, an image moving process S121 is performed. The procedure of the image moving process S121 is shown in Fig. 3.

In Fig. 3, designation of an eye movement position is first executed (S21). In this eye movement position designating process, as shown in Figs. 6A and 6B, the eye movement position is designated when performing the simulation by horizontally moving a field of view through the lens from the left position of Fig. 6A to the right position of Fig. 6B, for example. In Figs. 6A and 6B, it is preferable to designate the eye movement position in a simple method like designating the edge position of the right window RW on the books BK displayed on the right side with a cursor of a mouse included in the input unit 12.

Then, the standard mode or the watch mode is set in the image data control portion 169 through the input unit 12 (S22). For example, the standard mode is set in the image data control portion 169.

Then, a process of calling visual motion characteristic data from the recording unit 14 is executed (S23), and the movement amount of the processing image data 62 is calculated (S24).

In the process S24 of calculating the movement amount of processing image data, the movement amount of the processing image data 62 is calculated from the visual motion characteristic data called from the recording unit 14 and the movement amount designated in the eye movement position designating process.

For example, when the ratio between the rotation angle θ of the head and the rotation angle α of the eyes is θ : α = 4 : 6 and the screen is made to move entirely by an angle of 30°, the amount of rotation of the head becomes 30° x 0.4 = 12° in the right direction. Accordingly, the movement amount on a straight line of image data according to the rotation angle (12°) of the head is calculated. In general, the rotation angle of the head is large when the lens user is a head mover and is small when the lens user is an eye mover.

The processing image data 62 is made to move by the amount calculated in the processing image data movement amount calculating process S24 (S25). The processing image data 62 continues in order of Figs. 7A to 7C. When the lens user is a head mover, the processing image data 62 moves from Fig. 7A to Fig. 7C. However, when the lens user is an eye mover, the processing image data 62 moves from Fig. 7A only to Fig. 7B.

The processing image data 62 is displayed on the display unit 13 (S27) . In this case, for the processing image data 62 that is displayed on the display unit 13 through the lens, a way of being seen according to the optical characteristic of the progressive power lens in the processing image data 62 of Figs. 7A to 7C is image-processed by lens design data called from the recording unit 14 (S26), and the result is displayed.

For example, the progressive power lens of soft design is displayed in order of Figs. 8A to 8C. In Figs. 8A to 8C, the progressive power lens is displayed as an image in which clearly visible regions of the far vision portion and near vision portion are made relatively narrow and distortion of an image and astigmatism on the side are suppressed. In Figs. 8A to 8C, reference numeral 70 indicates the transmission position of a primary line of sight of a lens user, that is, the center of the line of sight. In Figs. 8B and 8C, the center 70 of the line of sight indicates the books BK.

Using Figs. 8A to 8C, a difference of simulation performed by the head mover and the eye mover will be described. In the case of the head mover, the head mover moves the head while maintaining the line of sight at the front until Fig. 8C in which the outside of the right window or the books BK are seen on the front. On the other hand, in the case of the eye mover, the rotation of eyes is mainly performed. Accordingly, in this case, the eye mover moves the head until Fig. 8B in the middle and tries to see the remaining part using a side portion of the lens by the rotation of the eyes. Thus, in the case of the eye mover, the books BK and a part of the inside of the right window RW are seen through the side portion of the design lens. Thus, a way of being seen for the head mover is a change from Fig. 8A to Fig. 8C, and a way of being seen for the eye mover is a change from Fig. 8A to Fig. 8B.

The progressive power lens of hard design is displayed in order of Figs. 9A to 9C. In Figs. 9A to 9C, the progressive power lens has larger astigmatism than that of soft design. Accordingly, a broken line 63 is displayed doubly in Figs. 9A to 9C. In Figs. 9A to 9C, reference numeral 70 indicates the transmission position of a primary line of sight of a lens user, that is, the center of the line of sight. In Figs. 9B and 9C, the center 70 of the line of sight indicates the books BK.

Similar to Figs. 8A to 8C, some of the books BK are seen through a central portion of the design lens in the case of the head mover and the books BK are seen through a side portion of the design lens in the case of the eye mover in Figs. 9A to 9C. Thus, a way of being seen for the head mover is a change from Fig. 9A to Fig. 9C, and a way of being seen for the eye mover is a change from Fig. 9A to Fig. 9B. The processing image data 62 before and after movement is stored in the recording unit 14.

The suitability of the head mover and the eye mover will be described on the basis of Figs. 8A to 9C.

In the case of the head mover, soft design with little sway shown in Figs. 8A to 8C is suitable since there is a large movement of the field of view. In the hard design shown in Figs. 9A to 9C, the sway caused by large distortion in the progressive portion is felt more seriously by the large movement of the field of view.

On the contrary, in the case of the eye mover, the hard design that makes wide the far vision portion and the near vision portion shown in Figs. 9A to 9C is advantageous in that an object or a scene can be clearly seen through a side portion only by slightly rotating the head and then rotating the eyes.
In addition, the small movement of the field of view is also effective in that it is difficult to feel the sway caused by the large distortion. In addition, the head mover and the eye mover are not classified clearly, but most lens users have both the elements in different ratios. In addition, not only progressive power lenses of clear hard design or clear soft design but also many intermediate type progressive power lenses having both the elements exist.

Returning to the mode setting S22 to set the watch mode in the image data control portion 169 through the input unit 12, the processing image data 62 is made to move according to the total value of the amount of rotation of the head and the amount of rotation of the eyes irrespective of the visual motion characteristic data. That is, even if the ratio between the rotation angle θ of the head and the rotation angle α of the eyes is θ : α = 4 : 6, the screen is made to move entirely by an angle of 30° like a perfect head mover. As a result, the processing image data 62 of Fig. 8C or Fig. 9C is displayed.

Returning to Fig. 2, it is selected whether to reproduce an operation history (S122). In case of reproducing the operation history, the image data control portion 169 is made to operate through the input unit 12. Then, the processing image data 62 before and after movement recorded in the recording unit 14, that is, images of Fig. 8A and Fig. 8B or 8C and images of Fig. 9A and Fig. 9B or 9C are displayed on the display unit 13 alternately and repeatedly. In case of stopping the reproduction of the operation history, the image data control portion 169 is made to operate through the input unit 12. In case of not reproducing the operation history, the operation of the image data control portion 169 is stopped through the input unit 12.

### Operations and effects of the present embodiment

The present embodiment is a simulation apparatus that is used for lens evaluation when performing lens design in consideration of visual motion characteristics of a head mover and an eye mover and that is used when selecting a lens suitable for a user's visual motion characteristic before the user purchases a lens, for example. According to the present embodiment described above, the following effects are obtained.

(1) The simulation apparatus 1 includes: the visual motion characteristic data acquiring portion 165 that acquires visual motion characteristic data regarding the motion of head and eyes when a lens user moves his or her eyes to various observation objects; the image data control portion 169 that controls the movement amount of the processing image data 62, which is moved by the image moving portion 168, on the basis of the visual motion characteristic data acquired in the visual motion characteristic data acquiring portion 165; and the display unit 13 that displays the processing image data 62. Accordingly, since both the processing image data 62 before movement and the processing image data 62 after movement are displayed on the display unit 13, a way of being seen through a lens corresponding to the visual motion characteristic of each lens user can be simulated.

(2) Since the visual motion characteristic data acquired in the visual motion characteristic data acquiring portion 165 is the ratio between the rotation angle θ of the head and the rotation angle α of the eyes of the lens user, a way of being seen through a lens corresponding to the visual motion characteristic of each lens user can be simulated.

(3) The image data control portion 169 has the standard mode for moving the processing image data 62 according to the rotation angle θ of the head and the watch mode for moving the processing image data 62 according to the total value of the rotation angle θ of the head and the rotation angle α of the eyes irrespective of the ratio between the rotation angle θ of the head and the rotation angle α of the eyes and is configured to select the modes. Therefore, a way of being seen through a lens corresponding to the head mover or the eye mover of the lens user can be simulated by setting the standard mode, and a way of being seen when the lens user moves the eyes to watch an object can be simulated irrespective of the head mover or the eye mover by setting the watch mode.

(4) Since data on two kinds of lenses including a lens with the wide far vision portion and / or the wide near vision portion and a lens with the narrow far vision portion and / or the narrow near vision portion is included, the lens user can check through the simulation apparatus which one of the progressive power lens of hard design and the progressive power lens of soft design is suitable.

(5) Since the process of reproducing an operation history is performed, both the processing image data 62 before movement and the processing image data 62 after movement can be repeatedly displayed on the display unit 13 on the basis of data input once.

### Modifications of the embodiment

In addition, the invention is not limited to the embodiment described above and includes the following modifications in the range where the advantage of the invention can be obtained.

In the embodiment, mode setting using two modes of the standard mode and the watch mode is performed. However, the combination of the standard mode and the watch mode automatically changing from the standard mode to the watch mode is also possible. That is, a daily common observation action for roughly recognizing an object by moving the eyes in the standard mode and then positioning the face to face the object in order to see the object clearly can be simulated by the combination. Explaining a display image in this case with reference to Figs. 8A to 8C, the image changes from Fig. 8A to Fig. 8B, and the image further changes to Fig. 8C after a pause.

In the above embodiment, a case where a ophthalmic lens is the progressive power lens has been described. However, in the invention, a single focus lens may also be used. In this case, it is not necessary to display the broken line 63 in the processing image data 62. Also in the case of the progressive power lens, it is not necessarily required to express the boundary between the far vision portion, the progressive portion, and the near vision portion and the portion, which cannot be used due to the optical distortion, with the broken line 63. The customer can check the difference of a way of being seen depending on a change in the area of each region even if there is no broken line 63.

In addition, although the processing image data 62 is made to move left and right (horizontally) in the above embodiment, the processing image data 62 may also be moved vertically or obliquely in the invention.

Moreover, the invention is configured not only as the simulation apparatus 1 described in the above embodiment but also as a simulation program causing a calculation unit, such as a computer, to function as the simulation apparatus 1 and recording media, such as a CD-ROM and a memory card, which records the simulation program so as to be readable by the calculation unit.

## Claims

1. A simulation apparatus (1) comprising:
a design data acquiring unit (163) that acquires lens design data;
a lens design unit (166) that designs a lens on the basis of the lens design data;
an image data acquiring unit (164) that acquires image data corresponding to a lens user's visual sense;
an image processing unit (167) that creates processing image data which is the image data seen through the design lens designed by the lens design unit; and a display unit (13) that displays the processing image data, **characterized in that** the simulation apparatus further comprises
a visual motion characteristic data acquiring unit (165) that acquires visual motion characteristic data regarding the motion of head and eyes when the lens user moves the eyes to various observation objects;
an image moving unit (168) that moves the processing image data created by the image processing unit;
an image data control unit (169) that controls a movement amount of the processing image data, which is moved by the image moving unit, on the basis of the visual motion characteristic data.

2. The simulation apparatus according to Claim 1, wherein the visual motion characteristic data is a ratio between an amount of rotation of the head and an amount of rotation of the eyes when the lens user moves the eyes to various observation objects.

3. The simulation apparatus according to Claim 1 or 2, wherein the image data control unit has a standard mode in which the processing image data is made to move according to the amount of rotation of the head acquired in the visual motion characteristic data acquiring unit.

4. The simulation apparatus according to any one of the preceding Claims,
wherein the image data control unit has a watch mode in which the processing image data is made to move according to a total value of the amount of rotation of the head and the amount of rotation of the eyes acquired in the visual motion characteristic data acquiring unit.

5. The simulation apparatus according to any one of the preceding Claims,
wherein the lens is a progressive refractive power lens including a far vision portion for seeing a far place, a near vision portion for seeing a near place, and a progressive portion that is located between the far vision portion and the near vision portion and in which the refractive power progressively changes.

6. The simulation apparatus according to Claim 5,
wherein the lens design unit designs two kinds of lenses including a lens with the wide far vision portion and / or the wide near vision portion and a lens with the narrow far vision portion and / or the narrow near vision portion.

7. A simulation program causing a calculation unit to function as the simulation apparatus according to any one of the preceding Claims.

8. A recording medium recorded with a simulation program in which the simulation program according to Claim 7 is recorded to be readable by the calculation unit.

## Patentansprüche

1. Simulationsvorrichtung (1), umfassend:
eine Gestaltungsdatenerfassungseinheit (163), die Linsengestaltungsdaten erfasst;
eine Linsengestaltungseinheit (166), die eine Linse auf Grundlage der Linsengestaltungsdaten gestaltet;
eine Bilddatenerfassungseinheit (164), die Bilddaten erfasst, die dem Sehsinn eines Linsenbenutzers entsprechen;
eine Bildbearbeitungseinheit (167), die Bearbeitungsbilddaten erzeugt, welche die Bilddaten bei Betrachtung durch die durch die Linsengestaltungseinheit gestaltete Gestaltungslinse sind; und
eine Anzeigeneinheit (13), welche die Bearbeitungsbilddaten anzeigt, **dadurch gekennzeichnet, dass** die Simulationsvorrichtung ferner umfasst:
eine Einheit (165) zum Erfassen charakteristischer Daten visueller Bewegung, die charakteristische Daten visueller Bewegung mit Bezug auf die Bewegung von Kopf und Augen, wenn der Linsenbenutzer die Augen zu verschiedenen Betrachtungsobjekten bewegt, erfasst;
eine Bildbewegungseinheit (168), welche die durch die Bildbearbeitungseinheit erzeugten Bearbeitungsbilddaten bewegt;
eine Bilddatensteuereinheit (169), die einen Bewegungsbetrag der Bearbeitungsbilddaten, die durch die Bildbewegungseinheit bewegt werden, auf Grundlage der charakteristischen Daten visueller Bewegung steuert.

2. Simulationsvorrichtung nach Anspruch 1,
bei der die charakteristischen Daten visueller Bewegung ein Verhältnis zwischen einem Betrag einer Drehung des Kopfes und einem Betrag einer Drehung der Augen, wenn der Linsenbenutzer die Augen zu verschiedenen Betrachtungsobjekten bewegt, sind.

3. Simulationsvorrichtung nach Anspruch 1 oder 2,
bei der die Bilddatensteuereinheit einen Standardmodus aufweist, in dem die Bearbeitungsbilddaten entsprechend dem in der Einheit zum Erfassen charakteristischer Daten visueller Bewegung erfassten Betrag der Drehung des Kopfes zur Bewegung gebracht werden.

4. Simulationsvorrichtung nach einem der vorhergehenden Ansprüche,
bei der die Bilddatensteuereinheit einen Beobachtungsmodus aufweist, in dem die Bearbeitungsbilddaten entsprechend einem in der Einheit zum Erfassen charakteristischer Daten visueller Bewegung erfassten Gesamtwert des Betrags der Drehung des Kopfes und des Betrags der Drehung der Augen zur Bewegung gebracht werden.

5. Simulationsvorrichtung nach einem der vorhergehenden Ansprüche,
bei der die Linse eine Linse mit progressiver Brechkraft ist, die einen Fernsichtabschnitt zum Sehen eines weit entfernten Orts, einen Nahsichtabschnitt zum Sehen eines nahen Orts und einen progressiven Abschnitt beinhaltet, der zwischen dem Fernsichtabschnitt und dem Nahsichtabschnitt angeordnet ist und in dem sich die Brechkraft progressiv ändert.

6. Simulationsvorrichtung nach Anspruch 5,
bei der die Linsengestaltungseinheit zwei Arten von Linsen gestaltet, einschließlich einer Linse mit dem breiten Fernsichtabschnitt und/oder dem breiten Nahsichtabschnitt und einer Linse mit dem engen Fernsichtabschnitt und/oder dem engen Nahsichtabschnitt.

7. Simulationsprogramm, das bewirkt, dass eine Berechnungseinheit als die Simulationsvorrichtung nach einem der vorhergehenden Ansprüche arbeitet.

8. Aufzeichnungsmedium, auf dem ein Simulationsprogramm aufgezeichnet ist, wobei das Simulationsprogramm nach Anspruch 7 so aufgezeichnet ist, dass es durch die Berechnungseinheit lesbar ist.

## Revendications

1. Appareil de simulation (1) comprenant :
une unité d'acquisition de données de conception (163) qui acquiert des données de conception de lentille ;
une unité de conception de lentille (166) qui conçoit une lentille sur la base des données de conception de lentille ;
une unité d'acquisition de données d'image (164) qui acquiert des données d'image correspondant au sens de la vue d'un utilisateur de lentille ;
une unité de traitement d'image (167) qui crée les données d'image de traitement qui sont les données d'image vues à travers la lentille de conception conçue par l'unité de conception de lentille ; et
une unité d'affichage (13) qui affiche les données d'image de traitement, **caractérisé en ce que** l'appareil de simulation comprend en outre
une unité d'acquisition de données caractéristiques de mouvement visuel (165) qui acquiert des données caractéristiques de mouvement visuel concernant le mouvement de la tête et des yeux lorsque l'utilisateur de la lentille déplace les yeux vers divers objets d'observation ;
une unité de déplacement d'image (168) qui déplace les données d'image de traitement créées par l'unité de traitement d'image ;
une unité de commande de données d'image (169) qui commande une quantité de mouvement des données d'image de traitement, qui est déplacée par l'unité de déplacement d'image, sur la base des données caractéristiques de mouvement visuel.

2. Appareil de simulation selon la revendication 1,
dans lequel les données caractéristiques de mouvement visuel sont un rapport entre une quantité de rotation de la tête et une quantité de rotation des yeux lorsque l'utilisateur de la lentille déplace les yeux vers divers objets d'observation.

3. Appareil de simulation selon la revendication 1 ou 2,
dans lequel l'unité de commande de données d'image comporte un mode standard dans lequel les données d'image de traitement sont amenées à se déplacer selon la quantité de rotation de la tête acquise dans l'unité d'acquisition de données caractéristiques de mouvement visuel.

4. Appareil de simulation selon l'une quelconque des revendications précédentes,
dans lequel l'unité de commande de données d'image possède un mode de surveillance dans lequel les données d'image de traitement sont amenées à se déplacer selon une valeur totale de la quantité de rotation de la tête et de la quantité de rotation des yeux acquise dans l'unité d'acquisition de données caractéristiques de mouvement visuel.

5. Appareil de simulation selon l'une quelconque des revendications précédentes,
dans lequel la lentille est une lentille à pouvoir de réfraction progressif incluant une portion de vision de loin permettant de voir un endroit éloigné, une portion de vision de près permettant de voir un endroit proche, et une portion progressive qui est placée entre la portion de vision de loin et la portion de vision de près et dans laquelle le pouvoir de réfraction change progressivement.

6. Appareil de simulation selon la revendication 5,
dans lequel l'unité de conception de lentille conçoit deux types de lentille incluant une lentille avec la portion de vision de loin large et/ou la portion de vision de près large et une lentille avec la portion de vision de loin étroite et/ou la portion de vision de près étroite.

7. Programme de simulation amenant une unité de calcul à fonctionner comme l'appareil de simulation selon l'une quelconque des revendications précédentes.

8. Support d'enregistrement sur lequel est enregistré un programme de simulation
dans lequel le programme de simulation selon la revendication 7 est enregistré pour pouvoir être vu par l'unité de calcul.
